(19)

Europäisches Patentamt

European Patent Office

Office européen des brevets

(11)  **EP 4 591 980 A1**

(12)  **EUROPEAN PATENT APPLICATION**
published in accordance with Art. 153(4) EPC

(43) Date of publication:
**30.07.2025  Bulletin 2025/31**

(21) Application number: **23868466.6**

(22) Date of filing: **07.09.2023**

(51) International Patent Classification (IPC):
**B01J 27/18** *(2006.01)*    **B01J 37/03** *(2006.01)*
**B01J 37/06** *(2006.01)*    **B01J 37/00** *(2006.01)*
**C07C 51/377** *(2006.01)*   **C07C 57/04** *(2006.01)*

(52) Cooperative Patent Classification (CPC):
(C-Sets available)
**B01J 37/06; B01J 27/18; B01J 27/1806;**
**B01J 37/00; B01J 37/009; B01J 37/03;**
**B01J 37/031; B01J 37/04; C07C 51/377**    (Cont.)

(86) International application number:
**PCT/KR2023/013441**

(87) International publication number:
**WO 2024/063412 (28.03.2024 Gazette 2024/13)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **19.09.2022  KR 20220118052**
**05.09.2023  KR 20230117559**

(71) Applicant: **LG Chem, Ltd.**
**Yeongdeungpo-gu**
**Seoul 07336 (KR)**

(72) Inventors:
• **BANG, Jungup**
**Daejeon 34122 (KR)**

• **KIM, Jaeyoung**
**Daejeon 34122 (KR)**
• **BYUN, Wonbae**
**Daejeon 34122 (KR)**
• **CHOI, Jae Soon**
**Daejeon 34122 (KR)**
• **KIM, Mahnjung**
**Daejeon 34122 (KR)**
• **SONG, Cheolock**
**Daejeon 34122 (KR)**
• **KIM, Mira**
**Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54)  **CATALYST FOR PREPARING UNSATURATED CARBOXYLIC ACIDS AND DERIVATIVES THEROF**

(57)    Provided is a catalyst for preparing an unsaturated carboxylic acid and a derivative thereof, the catalyst capable of preparing the unsaturated carboxylic acid and the derivative thereof in a high yield over a long period of time during a dehydration reaction of a hydroxycarboxylic acid or a derivative thereof.

[FIG. 1]

**(Cont. next page)**

(52) Cooperative Patent Classification (CPC): (Cont.)

C-Sets
**C07C 51/377, C07C 57/04**

**Description**

[Technical Field]

Cross-reference to Related Application

The present application is based on, and claims priority from, Korean Patent Application Nos. 10-2022-0118052 and 10-2023-0117559, filed on September 19, 2022 and September 5, 2023, respectively, the disclosures of which are hereby incorporated by reference herein in their entirety.

The present invention relates to a catalyst for preparing an unsaturated carboxylic acid and a derivative thereof.

[Background Art]

Acrylic acid is generally synthesized through a two-step oxidation reaction of propylene which is a petroleum-derived raw material. However, due to the rapid rise in crude oil prices and concerns about future depletion, research is conducted to obtain unsaturated carboxylic acids, such as acrylic acid, etc., from biomass raw materials.

Lactic acid can be mass-produced from starch through fermentation, and acrylic acid can be produced through a dehydration reaction of lactic acid. Metal phosphate, metal sulfate, or zeolite-based catalysts are mainly used in the dehydration reaction of lactic acid. Among them, metal phosphate has been studied a lot, but there is a problem of low yield of acrylic acid.

Specifically, International Patent Publication No. WO2011/052178 suggests a method of synthesizing unsaturated carboxylic acids or derivatives thereof from hydroxycarboxylic acids or derivatives thereof through a dehydration reaction using hydroxyapatite ($Ca_{10}(PO_4)_6(OH)_2$) or $Sr_{10}(PO_4)_6(OH)_2$ as a catalyst. However, when acrylic acid is synthesized from lactic acid using hydroxyapatite, the yield of acrylic acid is about 50% to about 70%, and in the case of using $Sr_{10}(PO_4)_6(OH)_2$ in which Ca of hydroxyapatite is replaced with Sr, the yield of acrylic acid is around 30% which is even lower.

Accordingly, there is a need for the development of a method or a catalyst capable of synthesizing unsaturated carboxylic acids such as acrylic acid, etc., or esters thereof in a high yield.

[Disclosure]

[Technical Problem]

There is provided a catalyst capable of preparing an unsaturated carboxylic acid and a derivative thereof in a high yield over a long period of time during the preparation of the unsaturated carboxylic acid and the derivative thereof by a dehydration reaction of a hydroxycarboxylic acid or a derivative thereof, and a method of preparing a carboxylic acid and a derivative thereof using the same.

[Technical Solution]

There is provided a catalyst for preparing an unsaturated carboxylic acid or a derivative thereof, the catalyst including two or more types of hydroxyapatite with different amounts of alkali metal in the crystal, in which phosphorus (P), calcium (Ca), and alkali metal (M) present on the catalyst surface satisfy the following conditions of (i) and (ii):

(i) a molar ratio of calcium to phosphorus (a molar ratio of Ca/P): 1.0 or more and 1.2 or less,
(ii) a total molar ratio of calcium and alkali metal (M) to phosphorus (a molar ratio of (Ca+M)/P): 1.2 or more and 1.6 or less.

There is also provided a method of preparing the catalyst for preparing an unsaturated carboxylic acid or a derivative thereof, the method including the step of adding a raw material of alkali metal to a mixture including hydroxyapatite and pyrophosphate, and then reacting the mixture under conditions of a temperature of 130°C or higher and 250°C or lower, and a pressure of 3 atm or more and 40 atm or less, in which the hydroxyapatite, the pyrophosphate, and the raw material of alkali metal are added in such amounts that phosphorus (P), calcium (Ca), and alkali metal (M) present on the surface of the catalyst finally prepared satisfy the conditions of (i) and (ii).

There is also provided a method of preparing an unsaturated carboxylic acid or a derivative thereof, the method including the step of dehydrating a hydroxycarboxylic acid or a derivative thereof in the presence of the catalyst for preparing the unsaturated carboxylic acid or the derivative thereof.

[Effect of the Invention]

When hydroxycarboxylic acids or derivatives thereof are dehydrated using a catalyst according to the present invention, unsaturated carboxylic acids and derivatives thereof can be prepared in a high yield over a long period of time.

[Brief Description of the Drawings]

FIG. 1 is a graph showing the results of analyzing a mixture (A) of hydroxyapatite (HAP) and calcium pyrophosphate (CaPP), which is used in the preparation of a catalyst according to Preparation Example 1, and the resulting catalyst (B) by X-ray diffraction (XRD);
FIG. 2 is a graph showing the result of XRD analysis of a catalyst prepared in Comparative Preparation Example 1;
FIG. 3a is an image of transmission electron microscope (TEM) of analysis site Point 1 in the catalyst prepared in Preparation Example 1, and FIG. 3b shows the results of elemental analysis by energy dispersive spectroscopy (EDS) at the corresponding site;
FIG. 4a is an image of TEM of analysis site Point 2 in the catalyst prepared in Preparation Example 1, and FIG. 4b shows the results of elemental analysis by EDS at the corresponding site; and
FIG. 5 shows the results of XPS elemental analysis according to etching time during elemental analysis on the surface of a catalyst of Comparative Preparation Example 2.

[Best Mode for Carrying Out the Invention]

The terms used in this description are just for explaining exemplary embodiments and it is not intended to restrict the present invention. The singular expression caninclude the plural expression unless it is differently expressed contextually. It must be understood that the term "include", "equip", or "have" in the present description is only used for designating the existence of characteristics taken effect, steps, components, or combinations thereof, and do not exclude the existence or the possibility of addition of one or more different characteristics, steps, components or combinations thereof beforehand.

The present invention can be variously modified and have various forms, and specific embodiments will be illustrated and described in detail as follows. However, it is not intended to limit the present invention to the specific examples and it must be understood that the present invention includes every modification, equivalents, or replacements included in the spirit and technical scope of the present invention.

Hereinafter, a catalyst for preparing an unsaturated carboxylic acid and a derivative thereof according to the present invention and a method of preparing the unsaturated carboxylic acid and the derivative thereof using the same will be described.

In the present invention, it was confirmed that during the preparation of a catalyst for preparing an unsaturated carboxylic acid and a derivative thereof, when a mixture of hydroxyapatite and pyrophosphate is used, and an alkali metal salt is added in an optimal content range, which are reacted under high-temperature and high-pressure conditions, the prepared catalyst includes two or more types of hydroxyapatite compounds with different amounts of the alkali metal in the hydroxyapatite crystals, and the content ratio of phosphorus (P), calcium (Ca), and alkali metal (M) on the catalyst surface is controlled to an optimal range, and as a result, it is possible to prepare an unsaturated carboxylic acid and a derivative thereof with a high conversion rate and a high yield during a dehydration reaction of a hydroxycarboxylic acid or a derivative thereof, thereby completing the present invention.

Specifically, the catalyst for preparing the unsaturated carboxylic acid or the derivative thereof according to the present invention includes two or more types of hydroxyapatite with different amounts of alkali metal in the crystal, in which phosphorus (P), calcium (Ca), and alkali metal (M) present on the catalyst surface satisfy the following conditions of (i) and (ii):

(i) a molar ratio of calcium to phosphorus (a molar ratio of Ca/P): 1.0 or more and 1.2 or less,
(ii) a total molar ratio of calcium and alkali metal (M) to phosphorus (a molar ratio of (Ca+M)/P): 1.2 or more and 1.6 or less.

With regard to the hydroxyapatite compound which is used as the catalyst for preparing the unsaturated carboxylic acid or the derivative thereof, the amount of alkali metal in the compound affects selectivity in the preparation of the unsaturated carboxylic acid and the derivative thereof. Specifically, when the amount of alkali metal in the catalyst, particularly, on the catalyst surface is low, selectivity decreases, whereas when the amount of alkali metal is high, selectivity is maintained.

Further, a molar ratio of calcium to phosphorus (a molar ratio of Ca/P) on the catalyst surface affects the yield of the unsaturated carboxylic acid and the derivative thereof, i.e., the conversion rate of acrylic acid. When the molar ratio of Ca/P is too high or too low, the yield decreases.

Further, when the unsaturated carboxylic acid or the derivative thereof are prepared, the selectivity and the conversion rate show a trade-off relationship with each other, and thus it is difficult to improve selectivity and the conversion rate at the same time.

Meanwhile, it is difficult to introduce a high amount of alkali metal into hydroxyapatite due to its crystal structural characteristics. In contrast, in the case of pyrophosphate, it is possible to introduce a relatively high amount of alkali metal into the crystal structure of pyrophosphate, as compared to hydroxyapatite. Further, pyrophosphate can be converted to hydroxyapatite using hydroxyapatite as a seed when a high-temperature and high-pressure reaction is performed in the presence of hydroxyapatite.

Accordingly, in the present invention, when a catalyst for preparing an unsaturated carboxylic acid or a derivative thereof is prepared, a mixture of hydroxyapatite and pyrophosphate is used, and an alkali metal salt is added in an optimal amount range, which are reacted under high temperature and high pressure conditions, thereby introducing the alkali metal into each of hydroxyapatite and pyrophosphate, and at the same time, converting, into hydroxyapatite, pyrophosphate in which a high amount of alkali metal is introduced into hydroxyapatite. As a result, the prepared catalyst can include hydroxyapatite, which is derived from the raw material hydroxyapatite and has a relatively low amount of alkali metal, and hydroxyapatite, which is derived from pyrophosphate and has a relatively high amount of alkali metal.

In addition, hydroxyapatite, which is derived from pyrophosphate and has a relatively high amount of alkali metal, can increase the amount of alkali metal on the catalyst surface. Accordingly, the selectivity and conversion rate can be further improved when preparing the unsaturated carboxylic acid and the derivative thereof by controlling the mixing ratio with Ca and P while increasing the amount of alkali metal on the catalyst surface.

Specifically, in the catalyst according to the present invention, the molar ratio of calcium to phosphorus (P) (molar ratio of Ca/P) on the catalyst surface can be 1.0 or more and 1.2 or less, more specifically, 1.0 or more, or 1.05 or more, or 1.1 or more, and 1.2 or less, or less than 1.2, or 1.15 or less.

Further, the total molar ratio of calcium and alkali metal to phosphorus on the catalyst surface (molar ratio of (Ca+M)/P) can be 1.2 or more and 1.6 or less, more specifically, 1.2 or more, or 1.3 or more, or 1.5 or more, and 1.6 or less, or less than 1.6, or 1.55 or less.

Further, the catalyst has a high amount of alkali metal present on the catalyst surface, which is 4% by weight or more, and 8% by weight or less, based on the total weight of elements present on the catalyst surface, more specifically, 4% by weight or more, or 5% by weight or more, or 6% by weight or more, or 6.5% by weight or more, and 8% by weight or less, or 7.5% by weight or less, or 7% by weight or less.

Meanwhile, in the present invention, the element content (atomic%, at%) by the atomic weights of elements including phosphorus, calcium, and alkali metal on the catalyst surface can be measured through XPS analysis. The specific measurement and calculation methods thereof will be explained in detail in Experimental Examples below.

From the element content (at%) of calcium (Ca), the element content (at%) of phosphorus (P), and the element content (at%) of alkali metal (M) which are calculated through the XPS analysis, the molar ratio of calcium to phosphorus on the catalyst surface can be obtained from the ratio of the element content of Ca (Ca at%)/the element content of P (P at%). Further, the total molar ratio of calcium and alkali metal to phosphorus can be obtained from the ratio of (Ca at%+ M at%)/(P at%).

Further, the amount (% by weight, wt%) of alkali metal present on the catalyst surface can be calculated from the element content (at%) calculated above.

For example, according to Equation 1 below, the element content (at%) by the atomic weight of each element is converted to the element amount (wt%) by weight, and from the converted element amount (wt%) by weight of each element, the relative amount (wt%) of alkali metal present on the catalyst surface can be calculated according to Equation 2 below.

Element amount by weight (wt%) = [(Element content by atomic weight of corresponding element × Atomic weight of corresponding element)/Σ (Element amount of each element measured on catalyst surface × atomic weight of each measured element)] × 100      [Equation 1]

For example, the element content of Ca (at%) can be converted to the element amount (wt%) by weight using Equation of [(at% of Ca × atomic weight of Ca) / Σ (at% of each measured element × atomic weight of each measured element)] × 100.

Amount of alkali metal (wt%) = [Amount by weight (wt%) converted from element content of alkali metal present on the catalyst surface/$\Sigma$ (Amount by weight (wt%) of each element present on the catalyst surface, converted from element content) ] $\times$ 100 [Equation 2]

Further, the catalyst surface is an XPS depth upon XPS analysis, and the catalyst surface of the preset invention refers to an area corresponding to a depth of 10 nm measured from the interface where the catalyst is in contact with the atmosphere toward the interior center of the catalyst. Since XPS depth can vary depending on measurement conditions and materials, XPS analysis can be performed while appropriately changing the XPS analysis conditions to enable analysis of the above-described catalyst surface area, or while cutting the catalyst surface from the interface where the catalyst is in contact with the atmosphere to a depth of 10 nm toward the interior center of the catalyst.

In addition to controlling the amount ratio of phosphorus, calcium, and alkali metal on the catalyst surface as described above, the catalyst according to the present invention further controls the amount of the above elements in the catalyst, thereby further improving the selectivity and conversion rate during preparation of an unsaturated carboxylic acid or a derivative thereof.

Specifically, the amount of alkali metal in the catalyst can be 2% by weight or more and 5% by weight or less, based on the total weight of the catalyst, more specifically, 2% by weight or more, or 2.5% by weight or more, or 2.6% by weight or more, or 3% by weight or more, or 3.2% by weight or more, and 5% by weight or less, or 4.5% by weight or less, 4% by weight or less, or 3.7% by weight or less, based on the total weight of the catalyst.

Further, the molar ratio of calcium to phosphorus (molar ratio of Ca/P) in the catalyst can be 1.3 or more and 1.5 or less, more specifically, 1.3 or more, or 1.35 or more, or 1.4 or more, and 1.5 or less, or 1.45 or less.

The total molar ratio of calcium and alkali metal (M) to phosphorus (molar ratio of (Ca+M)/P) in the catalyst can be 1.2 or more and 2 or less, more specifically, 1.2 or more, or 1.35 or more, or 1.5 or more, or 1.65 or more, and 2 or less, or 1.9 or less, or 1.8 or less, or 1.75 or less, or 1.7 or less.

Meanwhile, in the present invention, the amount of elements including phosphorus, calcium, and alkali metal in the catalyst (% by weight (wt%) based on the total weight of the catalyst) can be determined by inductively coupled plasma analysis (ICP), more specifically, by inductively coupled plasma-optical emission spectroscopy (ICP-OES). The specific measurement and calculation methods will be explained in detail in Experimental Examples below.

From the amount (wt%) of calcium (Ca), the amount (wt%) of phosphorus (P), and the amount (wt%) of alkali metal (M) in the catalyst, based on the total weight of the catalyst, which are calculated through the ICP analysis, the molar ratio of calcium to phosphorus (molar ratio of Ca/P) and the total molar ratio of calcium and alkali metal to phosphorus (molar ratio of (Ca+M)/P) in the catalyst can be calculated.

In addition, as described above, the catalyst according to the present invention includes two or more types of hydroxyapatite with different amounts of alkali metal in the crystals. Specifically, the catalyst can include a first hydroxyapatite in which the amount of alkali metal in the crystal is 0.8% by weight or less, or 0% by weight to 0.8% by weight, or more than 0% by weight and 0.8% by weight or less, and a second hydroxyapatite in which the amount of alkali metal in the crystal is 3% by weight to 5% by weight.

Meanwhile, in the present invention, the alkali metal can be sodium, potassium, etc., and among them, the alkali metal can be sodium, considering the excellent improvement effect.

As described above, the catalyst according to the present invention can achieve high selectivity and high conversion rate at the same time during the preparation of the unsaturated carboxylic acid or the derivative thereof, as the amount ratio of phosphorus, calcium, and alkali metal on the catalyst surface and the entire surface is controlled to an optimal range.

The above catalyst can be prepared by a preparation method including the step of adding a raw material of alkali metal to a mixture including hydroxyapatite (HAP) and pyrophosphate, and then reacting the mixture under conditions of a temperature of 130°C or higher and 250°C or lower and a pressure of 3 atm or higher and 40 atm or lower. At this time, the hydroxyapatite, the pyrophosphate, and the raw material of alkali metal are added in such amounts that phosphorus (P), calcium (Ca), and alkali metal (M) present on the surface of the catalyst finally prepared satisfy the conditions of (i) and (ii).

Unlike HAP or CaPP which is commonly used alone in the preparation of hydroxyapatite-based catalysts, a mixture of HAP and pyrophosphate is used in the present invention, and thus the catalyst finally prepared includes hydroxyapatites with different amounts of alkali metal in the crystal structure, specifically, HAP in which the alkali metal is rarely substituted and HAP with a high amount of alkali metal. As described, HAP with various concentrations of alkali metal is included, which is more advantageous in terms of selectivity and conversion rate upon preparing the unsaturated carboxylic acid or the derivative thereof.

As demonstrated in Experimental Examples below, when a catalyst is prepared using HAP only by treating with an alkali metal salt under high-temperature and high-pressure conditions, the alkali metal is hardly substituted or is substituted in a low amount in the crystal structure of HAP. In addition, when only CaPP is used, conversion to HAP was rarely achieved or converted at a low conversion rate. This is because HAP acts as a seed to facilitate the phase transition of CaPP in the process of converting CaPP to HAP.

Meanwhile, in the method of preparing the catalyst according to the present invention, the pyrophosphate can specifically include calcium pyrophosphate ($Ca_2P_2O_7$ CaPP), $Ca_2P_2O_7 \cdot 2H_2O$, $CaNa_2P_2O_7$, $CaHPO_4$, $CaHPO_4 \cdot 2H_2O$, $Ca(H_2PO_4)_2$, $Ca(H_2PO_4) \cdot 2H_2O$, $Na_6(PO_4)_2$, $Na_{10}(P_3O_{10})_2$, $Ca_3(PO_4)$, $Ca_8(HPO_4)_2(PO_4)_4 \cdot 5H_2O$, $Ca_8H_2(PO_4)_6 \cdot 3H_2O$, $Ca_3H_2(PO_4)_6 \cdot 5H_2O$, $Ca_9(PO_4)_6$, $K_6(PO_4)_2$, or $K_{10}(P_3O_{10})_2$, etc., and any one thereof or a mixture of two or more thereof can be used.

The pyrophosphate is converted to hydroxyapatite during a high-temperature and high-pressure reaction in the presence of hydroxyapatite. When hydroxyapatite is absent, conversion to hydroxyapatite is difficult, and conversion of pyrophosphate to hydroxyapatite requires the use of a larger amount of alkali metal salt and a long reaction time.

The mixing ratio of hydroxyapatite ($Ca_5(PO_4)_3(OH)$ or $Ca_{10}(PO_4)_6(OH)_2$, HAP) and pyrophosphate can be appropriately determined considering the amounts of Ca, P, and alkali metal in the catalyst finally prepared. For example, hydroxyapatite and pyrophosphate can be used at a mixing molar ratio of 1:9 to 9: 1.

In addition, as the raw material of the alkali metal, alkali metal-containing hydroxide, bicarbonate, or carbonate can be used. More specific examples can include $NaOH$, $KOH$, $NaHCO_3$, $Na_2CO_3$, $KCO_3$, etc., and any one thereof or a mixture of two or more thereof can be used.

The raw material of the alkali metal can be introduced in such an amount that the amount of alkali metal on the surface of the catalyst finally prepared, specifically, a total molar ratio of calcium and alkali metal to phosphorus (molar ratio of (Ca+M)/P) satisfies 1.2 or more and 1.6 or less. For example, the raw material of the alkali metal can be introduced in an amount of 0.1 mol to 5 mol per 1 mol of Ca.

Meanwhile, after introducing the raw material of the alkali metal, the reaction can be carried out at a temperature of 130°C or higher and 250°C or lower, and a pressure of 3 atm or higher and 40 atm or lower.

More specifically, the reaction can be carried out at a temperature of 130°C or higher, or 150°C or higher, and 250°C or lower, or 200°C or lower, and at a pressure of 3 atm or higher, or 5 atm or higher, and 40 atm or lower, or 20 atm or lower, or 10 atm or lower. When the reaction is carried out under the above temperature and pressure conditions, the occurrence of side reactions and overreactions can be suppressed, and the catalyst can be prepared in a high yield.

Further, a high-temperature and high-pressure reactor can be used for the reaction under the high-temperature and high-pressure conditions.

As a result of the above reaction, the catalyst is precipitated, which is obtained by filtration and washing.

The catalyst prepared by the above preparation method includes two or more types of hydroxyapatite compounds with different amounts of alkali metal in the hydroxyapatite crystals, and at the same time, the molar ratio of calcium and alkali metal to phosphorus (P) in the catalyst is controlled to an optimal range, thereby preparing the unsaturated carboxylic acid and the derivative thereof in a high yield over a long period of time during the dehydration reaction of a hydroxycarboxylic acid or a derivative thereof.

Accordingly, in the present invention, provided is a method of preparing an unsaturated carboxylic acid and a derivative thereof using the catalyst.

Specifically, the preparation method includes the step of dehydrating a hydroxycarboxylic acid or a derivative thereof in the presence of the catalyst for preparing the unsaturated carboxylic acid or the derivative thereof.

Specific examples of hydroxycarboxylic acid which is a raw material in the method of preparing the unsaturated carboxylic acid and the derivative thereof according to the present invention can include lactic acid, citric acid, 3-hydroxypropionic acid, 3-hydroxy-2-methylpropionic acid, 3-hydroxybutanoic acid, 3-hydroxy-2-methylbutanoic acid, or 2,3-dimethyl-3-hydroxybutanoic acid, etc. Derivatives such as salts thereof, esters thereof, or dimers thereof, etc. can be used.

The hydroxycarboxylic acid or the derivative thereof can be used in the form of an aqueous solution of being dissolved in water, or can be used in the form of a solution of being dissolved in a mixed solvent in which a hydrophilic organic solvent such as alcohol or ether is mixed with water.

At this time, the concentration of the hydroxycarboxylic acid or the derivative thereof is not particularly limited, but can be 20% by weight or more and 60% by weight or less considering efficiency.

More specifically, in the above preparation method, the hydroxycarboxylic acid can be lactic acid, and the unsaturated carboxylic acid can be acrylic acid.

In addition, the amount of the catalyst used during the dehydration reaction can be appropriately selected considering the type of reactant, reaction time, etc. For example, per 1 g of the catalyst, the hydroxycarboxylic acid or the derivative thereof can be introduced in an amount of 0.05 g or more and 3 g or less per hour, or more specifically, in an amount of 0.1 g or more and 1 g or less per hour, or in an amount of 0.5 g per hour.

Further, the dehydration reaction can be performed in a continuous or batch manner using a fixed bed reactor. More specifically, the dehydration reaction can be performed in a continuous manner in which the product is continuously prepared by charging the catalyst in a fixed bed reactor and by continuously supplying the reactants to the reactor for reaction.

When the reaction is performed in the continuous manner using the fixed bed reactor, an inert gas such as nitrogen, argon, or helium, etc. can be used as a carrier gas. The input amount of the carrier gas is not particularly limited and can be appropriately determined depending on reaction conditions, such as the input amount of reactants. For example, the carrier gas can be introduced in an amount of 5 ml/min or more and 500 ml/min or less per 1 g of the catalyst.

Further, the dehydration reaction can be performed at a temperature of 300°C or higher and 500°C or lower. More specifically, the dehydration reaction can be performed at a temperature of 300°C or higher, or 350°C or higher, or 360°C or higher, and 500°C or lower, or 450°C or lower, or 370°C or lower.

Further, the dehydration reaction can be performed at a pressure of 0.5 bar or more and 5 bar or less. More specifically, the dehydration reaction can be performed at a pressure of 0.5 bar or more, or 0.8 bar or more, and 5 bar or less, or 2 bar or less, and more specifically, under normal pressure (1 $\pm$ 0.2 bar) conditions.

In addition, the reactants, the hydroxycarboxylic acid or the derivative thereof, can be introduced at a weight hour space velocity (WHSV) of 0.05 $h^{-1}$ or more and 3 $h^{-1}$ or less, more specifically, at a WHSV of 0.05 $h^{-1}$ or more, or 0.1 $h^{-1}$ or more, or 0.3 $h^{-1}$ or more, and 3 $h^{-1}$ or less, or 1 $h^{-1}$ or less, or 0.8 $h^{-1}$ or less.

When the reaction temperature exceeds 500°C, the reaction pressure is less than 0.5 bar, or the supply rate of the reactants is less than a WHSV of 0.1 $h^{-1}$, the catalyst activity can excessively increase, and it is understood that a side reaction of hydrogenolysis can occur, and as a result, selectivity can be reduced. On the contrary, when the reaction temperature is lower than 300°C, the reaction pressure is more than 5 bar, or the supply rate of the reactants is more than a WHSV of 1 $h^{-1}$, the conversion rate decreases and other reaction conditions must be severely increased, and as a result, it is understood that the catalyst life is shortened and costs will increase during the separation and recovery step of the product.

Through the dehydration reaction described above, at least part of the hydroxycarboxylic acid or the derivative thereof is converted to the unsaturated carboxylic acid or the derivative thereof.

In the method of preparing the unsaturated carboxylic acid or the derivative thereof according to the present invention, the above-described catalyst is used to improve the conversion rate to unsaturated carboxylic acid, thereby preparing the unsaturated carboxylic acid and the derivative thereof in a high yield.

Hereinafter, preferred exemplary embodiments will be provided for better understanding of the present invention. However, the following exemplary embodiments are provided only for illustrating the present invention, but the amount of the present invention is not limited by the following exemplary embodiments.

**Preparation Example 1**

35 g of HAP and 65 g of CaPP were mixed and placed in a high-temperature, high-pressure reactor, then 500 ml of 1 M NaOH solution was added, and reaction was allowed for 4 hours under conditions of 150°C and 5 atm. The resulting white precipitate was filtered and washed to obtain a catalyst (yield: 90%).

**Preparation Example 2**

30 g of HAP and 70 g of CaPP were mixed and placed in a high-temperature, high-pressure reactor, then 500 ml of 1 M NaOH solution was added, and reaction was allowed for 4 hours under conditions of 150°C and 5 atm. The resulting white precipitate was filtered and washed to obtain a catalyst (yield: 90%).

**Preparation Example 3**

50 g of HAP and 50 g of CaPP were mixed and placed in a high-temperature, high-pressure reactor, then 500 ml of 1 M NaOH solution was added, and reaction was allowed for 4 hours under conditions of 150°C and 5 atm. The resulting white precipitate was filtered and washed to obtain a catalyst (yield: 90%).

**Comparative Preparation Example 1**

A catalyst was prepared in the same manner as in Example 1, except that only HAP was used without adding CaPP.

**Comparative Preparation Example 2**

A catalyst was prepared in the same manner as in Example 1, except that only CaPP was used without adding HAP.

**Comparative Preparation Example 3**

Only $Ca_{10-x}(PO_4)_{6-x}(HPO_4)_x(OH)_{2-x}$ (x=1) (molar ratio of Ca/P =1.5) was used as calcium-deficient hydroxyapatite (calcium deficient HAP, CDHAP) without adding CaPP, and placed in a high-temperature and high-pressure reactor, 500 ml of 1 M NaOH solution was added and reaction was allowed for 4 hours under conditions of 150°C and 5 atm. The resulting white precipitate was filtered and washed to obtain a catalyst (yield: 95%).

**Example 1**

A fixed bed reactor was filled with the catalyst prepared in Preparation Example 1, and a dehydration reaction was performed by supplying an aqueous solution of 30% by weight of lactic acid at a WHSV of 0.32 $h^{-1}$ at a reaction temperature of 360°C and normal pressure (1±0.2 bar).
During the first 4 hours of the reaction, which was a reaction stabilization time, the reactants were collected and removed, and a liquid product obtained for 2 hours to 4 hours was recovered as a liquid sample using a cooling collector at 4°C.

**Example 2**

It was performed in the same manner as in Example 1, except that a fixed bed reactor was filled with the catalyst prepared in Preparation Example 1, and a dehydration reaction was performed by supplying an aqueous solution of 40% by weight of lactic acid at a WHSV of 0.43 $h^{-1}$ at a reaction temperature of 370°C and normal pressure.

**Example 3**

It was performed in the same manner as in Example 1, except that a fixed bed reactor was filled with the catalyst prepared in Preparation Example 2, and a dehydration reaction was performed by supplying an aqueous solution of 30% by weight of lactic acid at a WHSV of 0.32 $h^{-1}$ at a reaction temperature of 360°C and normal pressure.

**Example 4**

It was performed in the same manner as in Example 1, except that a fixed bed reactor was filled with the catalyst prepared in Preparation Example 2, and a dehydration reaction was performed by supplying an aqueous solution of 60% by weight of lactic acid at a WHSV of 0.68 $h^{-1}$ at a reaction temperature of 380 and normal pressure.

**Example 5**

It was performed in the same manner as in Example 1, except that a fixed bed reactor was filled with the catalyst prepared in Preparation Example 3, and a dehydration reaction was performed by supplying an aqueous solution of 30% by weight of lactic acid at a WHSV of 0.32 $h^{-1}$ at a reaction temperature of 360°C and normal pressure.

**Comparative Examples 1 to 3**

It was performed in the same manner as in Example 1, except that the catalysts prepared in Comparative Preparation Examples 1 to 3 were used, respectively.

**Experimental Example 1**

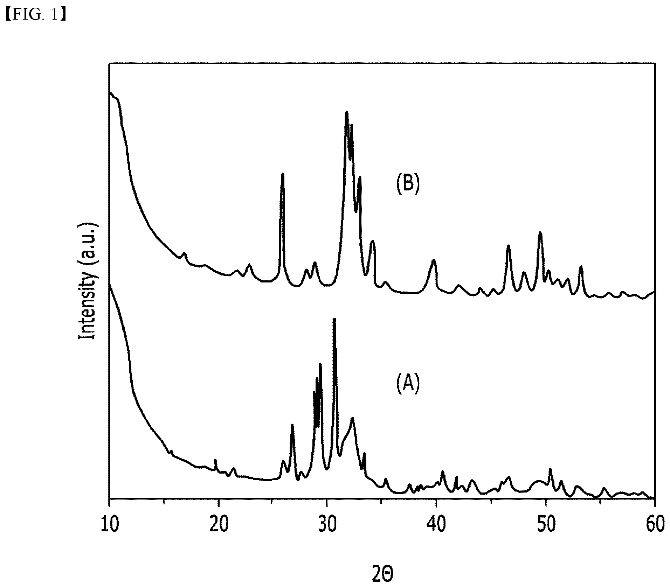

The mixture of HAP and CaPP used in preparing the catalyst according to Preparation Example 1 and the resulting catalyst were subjected to phase analysis using XRD, and the results are shown in FIG. 1.

<XRD analysis conditions>

Device used: XRD-D8_Advance (Powder XRD)

The sample powder was placed in the groove in the center of a general powder holder, and the surface was evenly prepared using a slide glass, with the height equal to the edge of the holder. The sample was measured in a 2theta ranging from 10° to 60° with a 0.017° step size and 3 sec/step using 1st Slit 2mm.
In FIG. 1, (A) shows the results of analyzing the mixture of hydroxyapatite (HAP) and calcium pyrophosphate (CaPP) which are used in the preparation of the catalyst according to Preparation Example 1, and (B) shows the results of analyzing the prepared catalyst.

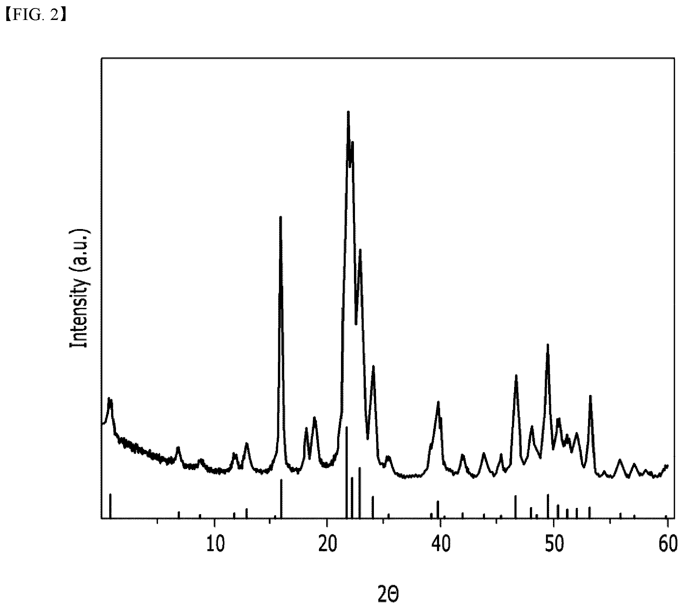

Further, the catalyst prepared in Comparative Preparation Example 1 was subjected to XRD analysis in the same manner as above, and the results are shown in FIG. 2.

The experimental results of FIGS. 1 and 2 confirmed that the catalyst of Preparation Example 1 consisted of hydroxyapatite compounds with different amounts of Na, whereas the catalyst of Comparative Preparation Example 1 consisted of a single phase of HAP with a high amount of Na.

**Experimental Example 2**

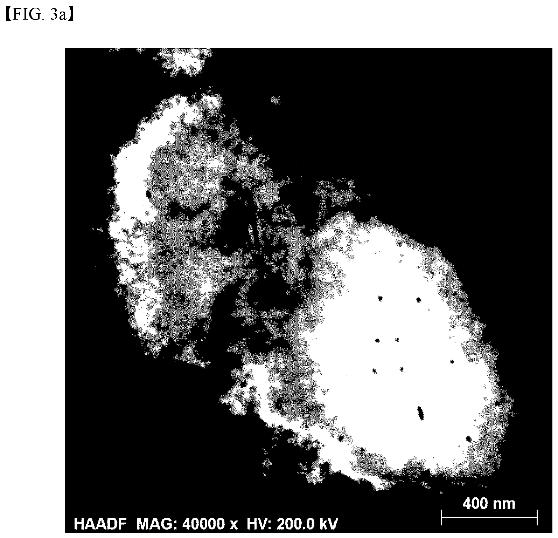

Two analysis sites (Point 1 and Point 2) were randomly determined for the catalyst prepared in Preparation Example 1, and spot energy dispersive spectroscopy (EDS) was performed for each using a transmission electron microscope (TEM) under the following conditions. The results are shown in FIGS. 3A to 4B, respectively.

<Analysis conditions>

Experiment equipment: Titan G2 80-200 Field Emission Transmission Electron Microscope
FE-TEM (Field Emission Transmission Electron Microscopy)
STEM-HAADF (Scanning transmission electron microscopy-High angle annular dark field imaging),
STEM-EDS (Energy dispersive X-ray spectroscopy) Spectrum
Experimental conditions: acceleration voltage 200 kV

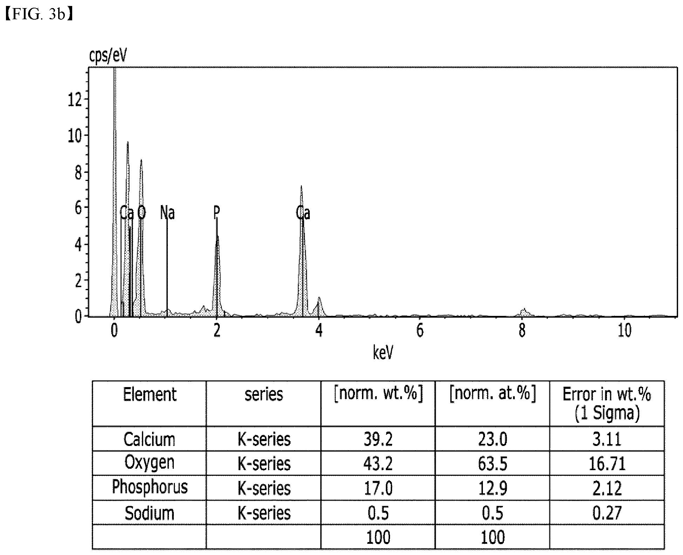

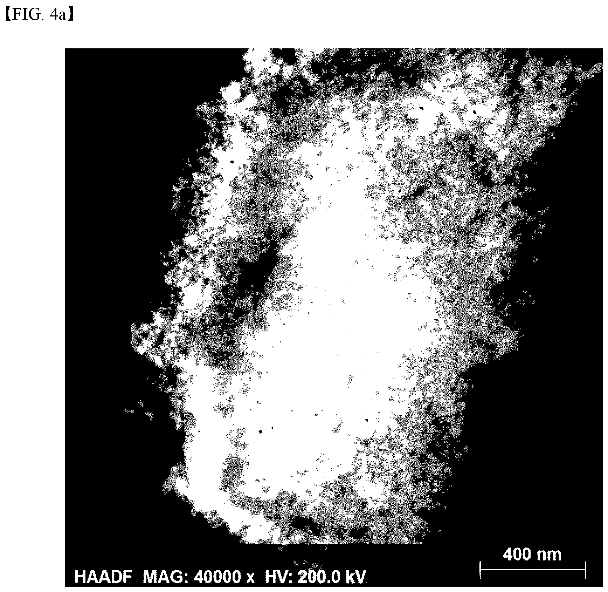

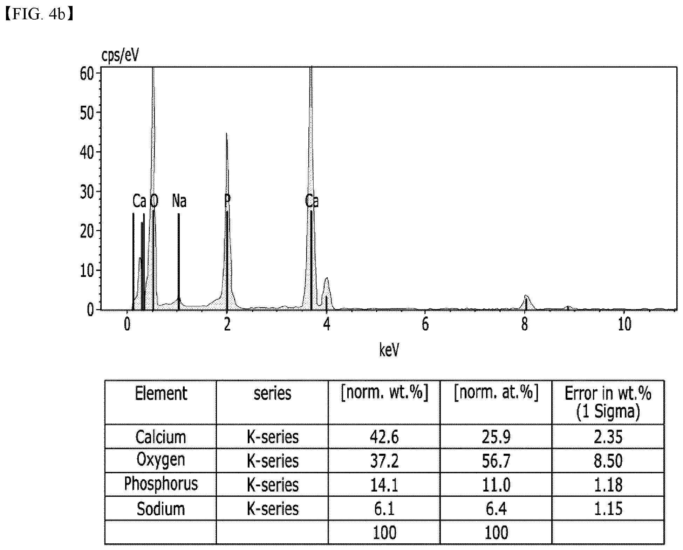

FIG. 3a is an image of TEM of analysis site Point 1 in the catalyst prepared in Preparation Example 1, and FIG. 3b shows the results of elemental analysis by EDS at the corresponding site. Further, FIG. 4a is an image of TEM of analysis site Point 2 in the catalyst prepared in Preparation Example 1, and FIG. 4b shows the results of elemental analysis by EDS at the corresponding site.

The above analysis results confirmed that the catalyst prepared according to the present invention includes at least two types of hydroxyapatite compounds with different amounts of alkali metal in hydroxyapatite crystals.

**Experimental Example 3**

1) Elemental analysis on catalyst surface

The surfaces of the catalysts prepared in the Preparation Examples and Comparative Preparation Examples were subjected to XPS analysis under the following conditions, and the results are shown in Table 1 below.

<XPS analysis method>

The amounts of elements on the catalyst surface were analyzed using an X-ray photoelectron spectroscopy (model name: Nexsa, manufacturer: Thermo Fisher Scientific Inc.) equipped with an Ar ion etching device, and calculated as a relative content ratio (atomic percent: atom%(at%)). In this regard, the catalyst surface refers to an area corresponding to a depth of 10 nm from the interface where the catalyst is in contact with the atmosphere toward the interior center of the catalyst. Accordingly, during the XPS surface analysis, while etching the catalyst surface using an etching method with Ar ions, specifically, using the Ar ion etching device provided in the photoelectron spectroscopy, at a rate of 1 nm to 3 nm per 10 seconds, the elemental distribution was analyzed by XPS.

In addition, during XPS analysis, monochromatic Al K$\alpha$ (1486.6 eV) was used as an X-ray source, and Shirley Peak background, ALTHERMO1 Sensitivity factor, TPP-2M Energy compensation factor were applied. Further, the amounts (atomic percent, based on the total atomic weights of elements present on the catalyst surface, at%) of elements present on the catalyst surface were calculated using Avantage software.

The elements present on the catalyst surface are Ca, P, Na, C, and O, and the calculation results of Ca, P, and Na are shown in Table 1 below.

In addition, the amount of Na present on the catalyst surface (based on the total weight of elements present on the catalyst surface, % by weight (wt%)) was calculated from the calculated element content (at%).

[Table 1]

| | Ca content (at%) | P content (at%) | Na content (at%) | Na amount on catalyst surface (wt%) | Molar ratio of Ca/P | Molar ratio of Ca+Na/P |
|---|---|---|---|---|---|---|
| Preparation Example 1 | 15 | 14 | 6 | 7 | 1.1 | 1.5 |
| Preparation Example 2 | 14 | 13 | 7 | 7 | 1.1 | 1.6 |
| Preparation Example 3 | 18 | 15 | 5 | 5 | 1.2 | 1.5 |
| Comparative Preparation Example 1 | 20 | 15 | 0.8 | 0.8 | 1.3 | 1.4 |
| Comparative Preparation Example 2 | 15 | 18 | 5 | 6 | 0.8 | 1.1 |
| Comparative Preparation Example 3 | 22 | 11 | 0.9 | 0.9 | 1.6 | 1.7 |

In the case of Comparative Preparation Example 1, which was prepared using only HAP without adding CaPP, the Na content on the catalyst surface was 0.8 at%. In contrast, in the case of Comparative Preparation Example 2, which was prepared using only CaPP without adding HAP, the Na content on the catalyst surface was 5 at%. The two catalysts had different contents of Na in hydroxyapatite. These results indicate that the catalysts of Preparation Examples 1 and 2, in which a mixture of HAP and CaPP was used, had both the hydroxyapatite compound with a low Na content of 0.8 atom%, derived from HAP, and the hydroxyapatite compound with a high Na content, derived from CaPP.

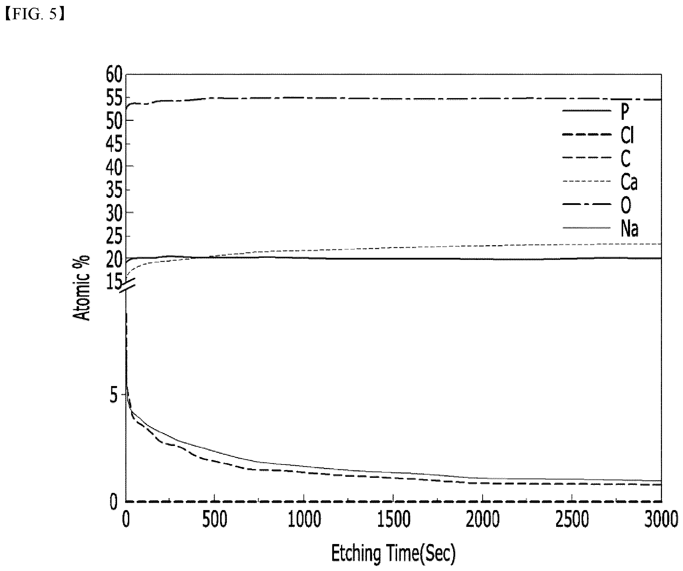

In addition, the results of XPS elemental analysis according to etching time, i.e., depth from the catalyst surface, upon analyzing elements on the surface of the catalyst of Comparative Preparation Example 2, are shown in FIG. 5.

As shown in FIG. 5, when the catalyst surface was analyzed while cutting, the concentration of Na on the surface of the catalyst was high, and the deeper inside the catalyst, the lower the Na concentration.

It is thought that the reason why the Na concentration on the catalyst surface is higher than the inside is the high surface Na amount of a hydrothermal reaction precursor CaPP, which influences the high Na amount during phase transition to the catalyst.

**2) Elemental analysis on catalyst**

Further, the catalysts prepared in the Preparation Examples and Comparative Preparation Examples were subjected to ICP analysis under the following conditions, and from the results, the amount of each element present in the catalysts (based on the total weight of the catalyst, wt%) is shown in Table 2 below.

In addition, the molar ratios of Ca/P and Ca+Na/P present in the catalyst were calculated from Ca, P and Na amounts in the catalyst (based on the total weight of the elements present on the catalyst surface, wt%), respectively, and shown in Table 2 below.

<ICP analysis conditions>

Device used: ICP-OES (Perkin Elmer, OPTIMA 7300DV)

The sample (0.01 g) was accurately weighed, and then dissolved using an acid. When the sample was clearly dissolved, ultrapure water was added and Sr as an internal standard was added. ICP-OES analysis was performed by keeping the final volume constant and adjusting the final concentration and measurement mode considering the sensitivity and concentration of the elements.

[Table 2]

| | Ca amount (wt%) | P amount (wt%) | Na amount (wt%) | Molar ratio of Ca/P | Molar ratio of Ca+Na/P |
|---|---|---|---|---|---|
| Preparation Example 1 | 35 | 19 | 3.7 | 1.4 | 1.7 |
| Preparation Example 2 | 35 | 19 | 3.2 | 1.4 | 1.7 |
| Preparation Example 3 | 37 | 20 | 2.6 | 1.4 | 1.5 |
| Comparative Preparation Example 1 | 39 | 19 | 0.3 | 1.6 | 1.6 |
| Comparative Preparation Example 2 | 34 | 26 | 0.9 | 1.0 | 1.1 |

(continued)

|  | Ca amount (wt%) | P amount (wt%) | Na amount (wt%) | Molar ratio of Ca/P | Molar ratio of Ca+Na/P |
|---|---|---|---|---|---|
| Comparative Preparation Example 3 | 38.4 | 17.5 | 0.4 | 1.7 | 1.7 |

**Experimental Example 4**

The reaction products obtained in the Examples and Comparative Examples were quantitatively analyzed using liquid chromatography (High Performance Liquid Chromatography (HPLC)), and the conversion rate (conversion, %) of hydroxycarboxylic acid or derivative thereof, acrylic acid selectivity (C%) and acetaldehyde selectivity (C%) were calculated using the analysis results according to the following Equations 3 to 5, respectively. At this time, the hydroxycarboxylic acid or derivative thereof in the Examples and Comparative Examples was lactic acid, and the experimental results are shown in Table 3 below.

<HPLC analysis conditions>

The reaction products obtained in the Examples and Comparative Examples were diluted 15-fold with distilled water, respectively and then quantitatively analyzed using HPLC (Agilent 1260 Infinity II).
- Eluent: 0.005 mol $H_2SO_4$ (aq)
- Eluent flow rate: 0.4 mL/min
- Column: Aminex HPX-87H
- Column temperature: 10°C
- Detector: UV 210~300 nm
- Analysis time: 70 min
- Analysis pressure: ~70 bar

[Equation 3]

$$\text{Conversion rate (\%) of hydroxycarboxylic acid or derivative thereof} = 100 \times (A\text{-}B)/A$$

in Equation 3,
A represents the moles of hydroxycarboxylic acid or derivative thereof before reaction,
B represents the moles of hydroxycarboxylic acid or derivative thereof after reaction.

[Equation 4]

$$\text{Acrylic acid selectivity (C\%)} = 100 \times (E/D) \times C_A$$

in Equation 4,
D represents the moles of reacted hydroxycarboxylic acid or derivative thereof, which corresponds to the value of (the moles of hydroxycarboxylic acid or derivative thereof before reaction - the moles of hydroxycarboxylic acid or derivative thereof after reaction),
E represents the moles of produced acrylic acid, and
$C_A$ represents the carbon selectivity of acrylic acid, which is 1.

[Equation 5]

$$\text{Acetaldehyde selectivity (C\%)} = 100 \times (F/D) \times C_{AD}$$

in Equation 5,
D represents the moles of reacted hydroxycarboxylic acid or derivative thereof, which corresponds to the value of (the moles of hydroxycarboxylic acid or derivative thereof before reaction - the moles of hydroxycarboxylic acid or derivative thereof after reaction),
F represents the moles of produced acetaldehyde, and
$C_{AD}$ represents the carbon selectivity of acetaldehyde, which is $2/3(\fallingdotseq 0.667)$.

[Table 3]

| | Moles of lactic acid before reaction (mmol) | Moles of lactic acid after reaction (mmol) | Moles of produced acrylic acid (mmol) | Moles of produced acetaldehyde (mmol) | Conversion rate (%) | Acrylic acid selectivity (C%) | Acetaldehyde selectivity (C%) |
|---|---|---|---|---|---|---|---|
| Example 1 | 14.2 | 0.1 | 9.5 | 2.5 | 99 | 67 | 12 |
| Example 2 | 19.1 | 0.4 | 12.2 | 3.4 | 98 | 65 | 12 |
| Example 3 | 14.2 | 0.1 | 9.3 | 3.2 | 99 | 66 | 15 |
| Example 4 | 28.4 | 0.9 | 16.8 | 5.0 | 97 | 61 | 12 |
| Example 5 | 14.2 | 0.1 | 9.5 | 2.5 | 95 | 64 | 13 |
| Comparative Example 1 | 14.2 | 1.3 | 6.8 | 5.0 | 91 | 49 | 24 |
| Comparative Example 2 | 14.2 | 4.7 | 5.7 | 1.6 | 67 | 60 | 11 |
| Comparative Example 3 | 14.2 | 0 | 6.0 | 2.7 | 100 | 42 | 19 |

As a result of the experiment, Examples 1 to 5, in which the catalysts of Preparation Examples 1 to 3 were used, showed a high acrylic acid selectivity of 61% or more, and a low acetaldehyde selectivity of 15% or less, together with a high conversion rate of 95% or more.

## Claims

1.  A catalyst for preparing an unsaturated carboxylic acid or a derivative thereof, the catalyst comprising two or more types of hydroxyapatite with different amounts of alkali metal in the crystal, wherein phosphorus (P), calcium (Ca), and alkali metal (M) present on the catalyst surface satisfy the following conditions of (i) and (ii):

    (i) a molar ratio of calcium to phosphorus: 1.0 or more and 1.2 or less,
    (ii) a total molar ratio of calcium and alkali metal to phosphorus: 1.2 or more and 1.6 or less.

2.  The catalyst for preparing the unsaturated carboxylic acid or the derivative thereof according to claim 1, wherein the amount of alkali metal present on the catalyst surface is 4% by weight or more, and 8% by weight or less, based on the total weight of elements present on the catalyst surface.

3.  The catalyst for preparing the unsaturated carboxylic acid or the derivative thereof according to claim 1, wherein the catalyst surface is an area corresponding to a depth of 10 nm as measured from the interface where the catalyst is in contact with the atmosphere toward the interior center of the catalyst.

4.  The catalyst for preparing the unsaturated carboxylic acid or the derivative thereof according to claim 1, wherein the amount of alkali metal in the catalyst is 2% by weight or more and 5% by weight or less, based on the total weight of the catalyst.

5.  The catalyst for preparing the unsaturated carboxylic acid or the derivative thereof according to claim 1, wherein the alkali metal is sodium or potassium.

6.  A method of preparing the catalyst for preparing the unsaturated carboxylic acid or the derivative thereof according to claim 1, the method comprising the step of adding a raw material of alkali metal to a mixture comprising hydroxyapatite and pyrophosphate, and then reacting the mixture under conditions of a temperature of 130°C or higher and 250°C or lower, and a pressure of 3 atm or more and 40 atm or less,
    Wherein the hydroxyapatite, the pyrophosphate, and the raw material of alkali metal are added in such amounts that phosphorus, calcium, and alkali metal present on the surface of the catalyst finally prepared satisfy the following conditions of (i) and (ii):

    (i) a molar ratio of calcium to phosphorus: 1.0 or more and 1.2 or less,
    (ii) a total molar ratio of calcium and alkali metal (M) to phosphorus: 1.2 or more and 1.6 or less.

7. The method of preparing the catalyst for preparing the unsaturated carboxylic acid or the derivative thereof according to claim 6, wherein the pyrophosphate is $Ca_2P_2O_7$, $Ca_2P_2O_7 \cdot 2H_2O$, $CaNa_2P_2O_7$, $CaHPO_4$, $CaHPO_4 \cdot 2H_2O$, $Ca(H_2PO_4)_2$, $Ca(H_2PO_4) \cdot 2H_2O$, $Na_6(PO_4)_2$, $Na_{10}(P_3O_{10})_2$, $Ca_3(PO_4)$, $Ca_8(HPO_4)_2(PO_4)_4 \cdot 5H_2O$, $Ca_8H_2(PO_4)_6 \cdot 3H_2O$, $Ca_3H_2(PO_4)_6 \cdot 5H_2O$, $Ca_9(PO_4)_6$, $K_6(PO_4)_2$, or $K_{10}(P_3O_{10})_2$.

8. The method of preparing the catalyst for preparing the unsaturated carboxylic acid or the derivative thereof according to claim 6, wherein the raw material of the alkali metal comprises NaOH, KOH, $NaHCO_3$, $Na_2CO_3$, $KCO_3$, or a mixture thereof.

9. A method of preparing an unsaturated carboxylic acid or a derivative thereof, the method comprising the step of dehydrating a hydroxycarboxylic acid or a derivative thereof in the presence of the catalyst for preparing the unsaturated carboxylic acid or the derivative thereof according to claim 1.

10. The method of preparing the unsaturated carboxylic acid or the derivative thereof according to claim 9, wherein the hydroxycarboxylic acid is lactic acid, and the unsaturated carboxylic acid is acrylic acid.

11. The method of preparing the unsaturated carboxylic acid or the derivative thereof according to claim 9, wherein the dehydration reaction is performed in a continuous manner using a fixed bed reactor.

12. The method of preparing the unsaturated carboxylic acid or the derivative thereof according to claim 9, wherein the dehydration reaction is performed by introducing the hydroxycarboxylic acid or the derivative thereof at a weight hour space velocity (WHSV) of $0.05\ h^{-1}$ or more and $3\ h^{-1}$ or less under conditions of a temperature of 300°C or higher and 500°C or lower and a pressure of 0.5 bar or more and 5 bar or less.

【FIG. 1】

EP 4 591 980 A1

【FIG. 2】

16

【FIG. 3a】

【FIG. 3b】

| Element | series | [norm. wt.%] | [norm. at.%] | Error in wt.% (1 Sigma) |
|---|---|---|---|---|
| Calcium | K-series | 39.2 | 23.0 | 3.11 |
| Oxygen | K-series | 43.2 | 63.5 | 16.71 |
| Phosphorus | K-series | 17.0 | 12.9 | 2.12 |
| Sodium | K-series | 0.5 | 0.5 | 0.27 |
| | | 100 | 100 | |

【FIG. 4a】

【FIG. 4b】

| Element | series | [norm. wt.%] | [norm. at.%] | Error in wt.% (1 Sigma) |
|---|---|---|---|---|
| Calcium | K-series | 42.6 | 25.9 | 2.35 |
| Oxygen | K-series | 37.2 | 56.7 | 8.50 |
| Phosphorus | K-series | 14.1 | 11.0 | 1.18 |
| Sodium | K-series | 6.1 | 6.4 | 1.15 |
| | | 100 | 100 | |

【FIG. 5】

## INTERNATIONAL SEARCH REPORT

| International application No. |
| --- |
| **PCT/KR2023/013441** |

### A. CLASSIFICATION OF SUBJECT MATTER

**B01J 27/18**(2006.01)i; **B01J 37/03**(2006.01)i; **B01J 37/06**(2006.01)i; **B01J 37/00**(2006.01)i; **C07C 51/377**(2006.01)i; **C07C 57/04**(2006.01)i

According to International Patent Classification (IPC) or to both national classification and IPC

### B. FIELDS SEARCHED

Minimum documentation searched (classification system followed by classification symbols)

B01J 27/18(2006.01); B01J 27/20(2006.01); B01J 29/82(2006.01); B01J 37/04(2006.01); B01J 37/08(2006.01); C07C 51/09(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 수산화인회석(hydroxyapatite), 피로인산(pyrophosphate), 아크릴산(acrylic acid), 젖산(lactic acid), 불포화 카르복실산(unsaturated carboxylic acid)

### C. DOCUMENTS CONSIDERED TO BE RELEVANT

| Category* | Citation of document, with indication, where appropriate, of the relevant passages | Relevant to claim No. |
| --- | --- | --- |
| X | KR 10-2012-0025888 A (KOREA RESEARCH INSTITUTE OF CHEMICAL TECHNOLOGY) 16 March 2012 (2012-03-16)<br>See paragraphs [0010]-[0058]; example 3; and claims 1-8. | 1-12 |
| A | KR 10-2022-0074721 A (LG CHEM, LTD.) 03 June 2022 (2022-06-03)<br>See entire document. | 1-12 |
| A | KR 10-2018-0118797 A (NOVOMER, INC.) 31 October 2018 (2018-10-31)<br>See entire document. | 1-12 |
| A | WO 2012-063044 A1 (LUCITE INTERNATIONAL UK LIMITED) 18 May 2012 (2012-05-18)<br>See entire document. | 1-12 |
| A | US 4729978 A (SAWICKI, R. A.) 08 March 1988 (1988-03-08)<br>See entire document. | 1-12 |

☐ Further documents are listed in the continuation of Box C.　　☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **14 December 2023** | **14 December 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2023/013441**

| Patent document cited in search report | | | Publication date (day/month/year) | Patent family member(s) | | | Publication date (day/month/year) |
|---|---|---|---|---|---|---|---|
| KR | 10-2012-0025888 | A | 16 March 2012 | KR | 10-1187804 | B1 | 09 October 2012 |
| KR | 10-2022-0074721 | A | 03 June 2022 | CN | 115427145 | A | 02 December 2022 |
| | | | | EP | 4108327 | A1 | 28 December 2022 |
| | | | | JP | 2023-520547 | A | 17 May 2023 |
| | | | | US | 2023-0159427 | A1 | 25 May 2023 |
| | | | | WO | 2022-114519 | A1 | 02 June 2022 |
| KR | 10-2018-0118797 | A | 31 October 2018 | AU | 2017-236914 | A1 | 08 November 2018 |
| | | | | BR | 112018069019 | A2 | 22 January 2019 |
| | | | | CA | 3018208 | A1 | 28 September 2017 |
| | | | | CN | 108884000 | A | 23 November 2018 |
| | | | | CN | 109153628 | A | 04 January 2019 |
| | | | | CN | 109153628 | B | 26 April 2022 |
| | | | | CN | 114736111 | A | 12 July 2022 |
| | | | | CO | 2018010877 | A2 | 22 October 2018 |
| | | | | EP | 3433222 | A1 | 30 January 2019 |
| | | | | EP | 3433222 | A4 | 06 November 2019 |
| | | | | EP | 3433222 | B1 | 08 March 2023 |
| | | | | EP | 3433226 | A1 | 30 January 2019 |
| | | | | EP | 3433226 | A4 | 06 November 2019 |
| | | | | EP | 3433226 | B1 | 15 December 2021 |
| | | | | JP | 2019-509307 | A | 04 April 2019 |
| | | | | JP | 2019-512527 | A | 16 May 2019 |
| | | | | JP | 6997459 | B2 | 04 February 2022 |
| | | | | KR | 10-2018-0128018 | A | 30 November 2018 |
| | | | | MX | 2018011139 | A | 21 November 2018 |
| | | | | MX | 2018011485 | A | 14 March 2019 |
| | | | | SA | 518400053 | B1 | 19 April 2022 |
| | | | | SA | 518400104 | B1 | 22 June 2022 |
| | | | | TW | 201802061 | A | 16 January 2018 |
| | | | | US | 10662139 | B2 | 26 May 2020 |
| | | | | US | 2017-0267618 | A1 | 21 September 2017 |
| | | | | US | 2020-0325093 | A1 | 15 October 2020 |
| | | | | US | 2021-0139402 | A1 | 13 May 2021 |
| | | | | WO | 2017-165323 | A1 | 28 September 2017 |
| | | | | WO | 2017-165344 | A1 | 28 September 2017 |
| | | | | ZA | 201807001 | B | 31 July 2019 |
| WO | 2012-063044 | A1 | 18 May 2012 | AU | 2011-327947 | A1 | 09 May 2013 |
| | | | | BR | 112013010735 | A2 | 09 August 2016 |
| | | | | BR | 112013010735 | B1 | 24 April 2019 |
| | | | | CA | 2814592 | A1 | 18 May 2012 |
| | | | | CA | 2814592 | C | 04 May 2021 |
| | | | | CN | 103209764 | A | 17 July 2013 |
| | | | | CN | 103209764 | B | 20 April 2016 |
| | | | | EP | 2637784 | A1 | 18 September 2013 |
| | | | | EP | 2637784 | B1 | 18 May 2022 |
| | | | | ES | 2922641 | T3 | 19 September 2022 |
| | | | | JP | 2014-508101 | A | 03 April 2014 |
| | | | | JP | 5843875 | B2 | 13 January 2016 |
| | | | | KR | 10-1834100 | B1 | 02 March 2018 |
| | | | | KR | 10-1896646 | B1 | 07 September 2018 |

Form PCT/ISA/210 (patent family annex) (July 2022)

## INTERNATIONAL SEARCH REPORT
### Information on patent family members

| International application No. |
| --- |
| **PCT/KR2023/013441** |

| Patent document cited in search report | Publication date (day/month/year) | Patent family member(s) | | Publication date (day/month/year) |
| --- | --- | --- | --- | --- |
| | | KR 10-2013-0132850 | A | 05 December 2013 |
| | | RU 2013126659 | A | 20 December 2014 |
| | | RU 2582603 | C2 | 27 April 2016 |
| | | SG 189440 | A1 | 31 May 2013 |
| | | TW 201228726 | A | 16 July 2012 |
| | | TW I547312 | B | 01 September 2016 |
| | | US 2015-0307437 | A1 | 29 October 2015 |
| | | US 2016-0228857 | A1 | 11 August 2016 |
| | | US 9346739 | B2 | 24 May 2016 |
| | | US 9782756 | B2 | 10 October 2017 |
| US 4729978 A | 08 March 1988 | None | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220118052
- KR 1020230117559

- WO 2011052178 A